# EUROPEAN PATENT SPECIFICATION

(11) **EP 1 670 404 B1**
(45) Date of publication and mention of the grant of the patent: **24.12.2008**
(21) Application number: 04775499.9
(22) Date of filing: 04.10.2004
(51) Int. Cl.: A61F 13/15, A61L 15/46, A61L 15/60

(54) **ABSORBENT ARTICLE COMPRISING AN ABSORBENT STRUCTURE**
SAUGFÄHIGER ARTIKEL MIT EINER SAUGFÄHIGEN STRUKTUR
ARTICLE ABSORBANT COMPRENANT UNE STRUCTURE ABSORBANTE

(30) Priority: 06.10.2003 SE 0302634
(43) Date of publication of application: 21.06.2006
(73) Proprietor: SCA Hygiene Products AB, 405 03 Göteborg (SE)
(72) Inventor: VARTIAINEN, Kent, S-443 92 Lerum (SE)
(74) Representative: Romare, Laila Anette
(86) International application number: PCT/SE2004/001405
(87) International publication number: WO 2005/032443

(56) References cited:
- WO-A1-01/41687
- WO-A1-91/11978
- US-A1- 2003 083 630
- US-A1- 2003 225 384
- US-B1- 6 387 495
- US-B1- 6 462 252
- US-B1- 6 649 806

## Description

### TECHNICAL FIELD

The present invention relates to an absorbent article such as a diaper, an incontinence pad, a sanitary towel or the like, the article having a liquid-permeable upper surface and comprising an absorbent structure.

### BACKGROUND ART

Common problems encountered when absorbent articles such as diapers, sanitary towels, incontinence pads or the like are used are that the use of such articles can lead to undesirable side effects such as skin irritation and problems of unpleasant odour.

Several undesirable side effects can arise as a consequence of or in connection with a pH increase. One example of such an undesirable side effect is irritative contact dermatitis. Another example of an undesirable side effect is the activity of enzymes such as lipases and proteases, the activity of which is greatly pH-dependent and increases as the pH increases. The skin begins to break down and becomes sensitive to mechanical action and bacterial attack. Another example of an undesirable side effect is that some bacteria, for example Proteus, can metabolize substances in urine and give rise to substances with an unpleasant odour such as ammonia and amines. At a high pH, the equilibrium of many odorous substances is shifted in such a way that more volatile components are formed, which leads to them smelling more than at a low pH.

Microorganisms which give rise to problems are of various kinds. Examples of microorganisms which cause odours and those which involve a risk of urinary tract infections are Proteus mirabilis, Proteus vulgaris, Escherichia coli, Enterococcus and Klebsiella. Examples of microorganisms which cause skin problems are Candida albicans, Staphylococcus sp. and Streptococcus sp.

It is known that a low pH is advantageous as far as reducing the occurrence of negative effects on the skin is concerned. US 3 794 034 describes the significance of pH in an absorbent article. In US 3 794 034, articles are impregnated with buffering substances, the pH being kept between 3.5 and 6.0.

From patent application SE 9702298-2, it is known to make use of an absorbent article which comprises a pH-regulating substance in the form of a partly neutralized superabsorbent material where the pH in the article after wetting is between 3.5 and 4.9. An absorbent article according to SE 9702298-2 leads to reduced risk of skin irritations and problems of unpleasant odour. A conventional superabsorbent material has a higher degree of neutralization than a superabsorbent material with a pH-regulating effect.

### DISCLOSURE OF INVENTION

By means of the present invention, an article has been produced which reduces the risk of odour nuisance and skin irritation at the same time as the article has a sufficient overall absorption capacity.

According to the invention, this has been achieved by virtue of the fact that a first superabsorbent material is an odour-inhibiting and/or bacteria-inhibiting superabsorbent material, and that the first superabsorbent material has a higher absorption rate than a second superabsorbent material. When the initial wetting takes place, it is primarily the odour-inhibiting and/or bacteria-inhibiting superabsorbent material with the higher absorption rate which takes up liquid. On subsequent wetting/wettings, it is instead mainly the second superabsorbent material which takes up the liquid. The second superabsorbent material absorbs liquid more slowly and/or starts to absorb only a certain time after the liquid reaches the material. One advantage of the invention is that the liquid which reaches the article first and is thus also in the article for the longest time is taken up mainly by the odour-inhibiting and/or bacteria-inhibiting superabsorbent material.

Another advantage of the invention is that the price of the superabsorbent material per article is in all likelihood lower than the price of the superabsorbent material for an article which contains only an odour/bacteria-inhibiting superabsorbent material. The diapers, sanitary towels and incontinence pads to which the invention relates are disposable products which are thrown away after use. Users of such disposable products use a number of products every day. It is therefore of considerable significance that the price per product is kept down. Today, the price of odour/bacteria-inhibiting superabsorbent material is higher than the price of conventional superabsorbent material.

A further advantage of the invention is that it is possible to obtain a greater overall absorption capacity than it is with an article which contains only the odour/bacteria-inhibiting superabsorbent material. This is due to the fact that a superabsorbent material which has a pH-lowering effect with a consequent odour/bacteria-inhibiting effect usually has a lower overall absorption capacity than a conventional superabsorbent without any pH-lowering effect.

According to one embodiment, the first superabsorbent material has a degree of neutralization of from 20 to 60%. Such a superabsorbent material has a lower pH than a conventional superabsorbent material.

According to one embodiment, the absorbent structure has a pH in the wet state during use which lies in the range 3.5-5.5. When the pH in the absorbent structure in the wet state lies in the range 3.5-5.5, the risk of undesirable odour and bacterial growth is reduced.
According to a similar embodiment, the absorbent structure has a pH in the wet state during use which lies in the range 3.5-4.9. It has been found that if the absorbent structure brings about a pH in the range 3.5-4.9, an appreciable growth-inhibiting effect on undesirable microorganisms is obtained in the article. The growth-inhibiting effect is based on the fact that many microorganisms have an activity which is greatly pH-dependent and decreases as the pH decreases. A lowering of the pH leads to reduced activity of the majority of microorganisms, which in turn leads to a reduction in unpleasant odour and negative effects on the skin in the form of skin irritation and primary or secondary skin infections as well as a reduced general risk of infection.

According to one embodiment, the second superabsorbent material has a degree of neutralization which is higher than 60%.

According to one embodiment, the first superabsorbent material has a greater specific surface per gram of superabsorbent material than the second superabsorbent material. By virtue of the fact that the first superabsorbent material and the second superabsorbent material have different geometrical shapes, for example, it is possible to obtain a first superabsorbent material which has a greater specific surface than the second superabsorbent material. For example, the first superabsorbent material can have a surface which is rough, for example superabsorbent particles which have a raisin-like surface. Such a superabsorbent material has a greater specific surface than a superabsorbent material of the same particle size but with a relatively smooth surface. In order to obtain different specific surfaces per gram of superabsorbent material, it is also possible for the first superabsorbent material to have a smaller particle size than the second superabsorbent material. For measuring the specific surface of the superabsorbents, use can be made of, for example, the B.E.T. method. The B.E.T. method is described in detail in EP 0 872 491 A1.

According to one embodiment, at least one superabsorbent material is treated in such a way that a difference in the absorption rate of the superabsorbent materials is obtained. For example, the second superabsorbent material is surrounded by a covering which is only slowly dissolved in and/or penetrated by the liquid which is to be absorbed, so that the superabsorbent material does not start to absorb liquid and swell to any great extent until the covering has been dissolved and/or penetrated by the liquid. The covering for the superabsorbent material with retarded activation time consists of, for example, gelatine, microcrystalline cellulose, cellulose derivative or a surfactant coating.

Another example for bringing about a difference in the absorption rate of the superabsorbent materials is to use a second superabsorbent material with a thermoreversible liquid take-up capacity such as, for example, a cross-linked polymer of N-isopropyl acrylamide. The liquid take-up capacity of the thermoreversible superabsorbent material is lower at a temperature above 32-35°C than the liquid take-up capacity at a temperature below 32-35°C. This means that the liquid is initially, that is to say at a temperature around 37°C, taken up primarily by the first superabsorbent material while, when it has cooled a few degrees, the liquid is also taken up by the second superabsorbent material. In order to achieve the thermoreversible liquid take-up capacity, it is also possible to copolymerize the second superabsorbent material with N-isopropyl acrylamide.

Other ways of bringing about a difference in the absorption rate of the superabsorbent materials are to use various cross-linkers or to neutralize the superabsorbent materials with various sails during manufacture.

According to one embodiment, the first superabsorbent material and the second superabsorbent material are distributed uniformly throughout the absorbent structure; for example, the superabsorbents are homogeneously mixed with cellulose fluff pulp. It is also possible for the first superabsorbent material and the second superabsorbent material to be mixed in such a way that at least some of the first superabsorbent material adheres to the second superabsorbent material.

According to another embodiment, the article has in the longitudinal direction a front portion, a rear portion and a crotch portion, the first superabsorbent material being mainly located in the crotch portion and the second superabsorbent material being mainly located in the front portion and the rear portion. When the wearer sits, stands or walks, the wetting point is in the crotch portion of the article. This means that the discharged liquid rapidly reaches the first superabsorbent material with the odour-inhibiting and/or bacteria-inhibiting effect. By virtue of the fact that the first superabsorbent material also absorbs liquid rapidly, the risk of leakage is moreover small.

According to a further embodiment, the first superabsorbent material is mainly located in the rear portion and the second superabsorbent material is mainly located in the front portion and the crotch portion of the article. When the wearer lies down, the wetting point is located further back in the article. This means that the discharged liquid rapidly reaches the first superabsorbent material with the odour/bacteria-inhibiting effect. By virtue of the fact that the first superabsorbent material also absorbs liquid rapidly, the risk of leakage is moreover small. For articles which are used at night or for people confined to bed, it is therefore an advantage that the first superabsorbent material is located in the rear portion.

There are also other possibilities for designing the absorbent structure so that the liquid reaches the first superabsorbent material before the second superabsorbent material. For example, the absorbent structure can have liquid-conveying ducts in the direction of the area which comprises the first superabsorbent material.

According to another embodiment, the absorbent structure has a first part which faces the liquid-permeable surface of the article and a second part which faces away from the liquid-permeable surface of the article, the first superabsorbent material being mainly located in the first part and the second superabsorbent material being mainly located in the second part. One advantage of such an embodiment is that the liquid reaches the first superabsorbent material with the odour/bacteria-inhibiting effect first.

The first part preferably consists of a receiving layer, which layer can rapidly receive a large quantity of liquid discharged in a short time. For example, the receiving layer consists of a fibrous layer comprising polyacrylate-based particles or a polyacrylate-based coating bonded to the fibrous layer. The bonding of the polyacrylate-based particles or the polyacrylate-based coating to the fibrous layer is effected by, for example, acrylic acid monomer being sprayed onto the fibrous layer, after which the acrylic acid monomer is allowed to polymerize. An example of such a material is a nonwoven material made of, for example, polyester. Drops of acrylic acid monomer are sprayed onto the nonwoven material, the acrylic acid monomer then being allowed to polymerize. In addition to functioning as liquid-absorbing material, the polymerized polyacrylic acid particles formed also function as a bonding agent. By means of hydrogen bonds between oxygen in the carboxylic groups of the acrylic acid and hydrogen in the polyester wadding, the receiving layer can be kept in a compressed state in the dry state. When wetting takes place, the hydrogen bonds present are broken, the material then expanding to its uncompressed thickness. The material subsequently swells further on account of the superabsorbent particles swelling when absorption of liquid takes place. This results in a material which is thin and relatively firmly compressed in the dry state but which has a great amount of free volume and high permeability when the material is then wetted. Another advantage of such a receiving layer is that the superabsorbent particles bind the liquid which is not drained by a storage layer, the risk of the surface next to the wearer becoming wet after initial wetting then being reduced. The embodiment also covers other ways of bonding a superabsorbent material to a fibrous structure.

According to one embodiment, the receiving layer is a superabsorbent foam, for example a polyacrylate-based foam. A polyacrylate-based superabsorbent foam is produced by a solution which consists of at least monomer, cross-linker, initiator and surfactant being saturated and pressurized with carbon dioxide in a vessel while being stirred. When the solution is removed from the vessel through a nozzle, the solution expands and a foamed structure is obtained. The foamed structure is then locked by polymerization and cross-linking is initiated by, for example, UV radiation. Finally, the material is compressed and dried.

The second part of the absorbent structure constitutes the liquid-storing part of the structure and can consist of one or more layer(s), at least one layer comprising the second superabsorbent material. It is of course also possible for the absorbent structure to comprise other superabsorbent material in addition to the first and the second superabsorbent material.

According to an example, the second part consists of two different layers. The first storage layer preferably has a higher content of superabsorbent material than the second storage layer. The second storage layer lies, for example, against the liquid-impermeable backing layer. The second storage layer also preferably has a greater extent than the first storage layer in the plane of the article. The second storage layer therefore functions as an extra safety zone, that is to say it takes up any liquid which ends up outside the first storage layer or outside the receiving layer.

According to a further embodiment, the first superabsorbent material is located in an absorbent insert.

It is of course also important that the superabsorbent materials function satisfactorily as far as properties such as, for example, permeability, absorption capacity under loading, spreading capacity and overall absorption capacity are concerned. For example, it is important that at least the first superabsorbent material is relatively permeable. Permeability of superabsorbent materials can be measured using, for example, the Saline Flow Conductivity (SFC) method. The method is described in detail in EP0752892A1.

### DESCRIPTION OF THE FIGURES

- Figure 1: shows a diaper according to the invention, seen from the side which is intended to lie against the wearer during use;
- Figure 2: shows a cross section along the line II-II through the diaper shown in Figure 1;
- Figure 3: shows a section through an alternative absorbent structure in the longitudinal direction of the absorbent structure;
- Figure 4: shows a section through a further alternative absorbent structure in the longitudinal direction of the absorbent structure, and
- Figure 5: shows a section through a further alternative absorbent structure in the longitudinal direction of the absorbent structure.

### DETAILED DESCRIPTION OF THE FIGURES

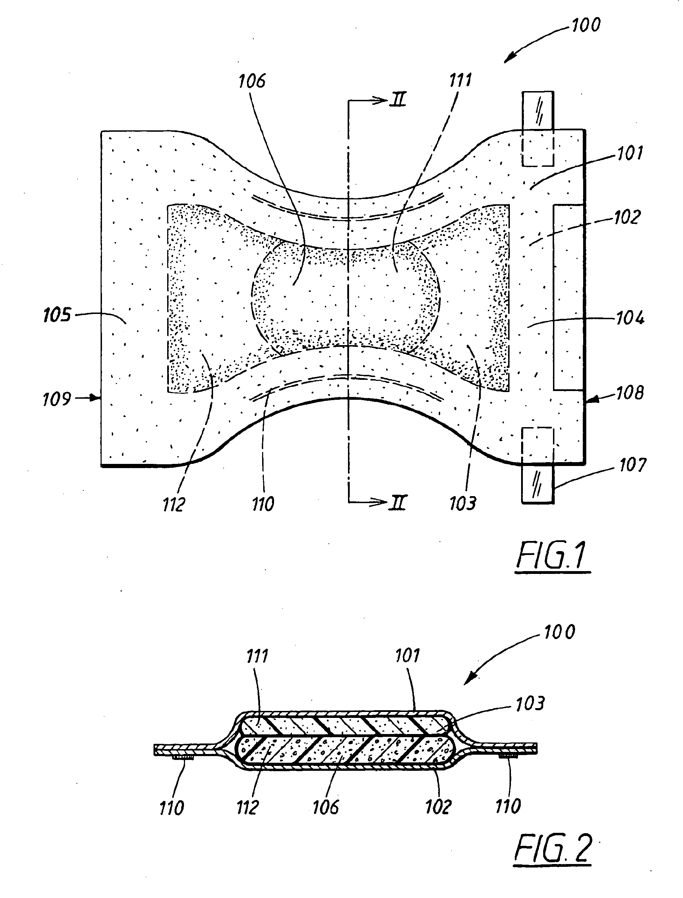

The diaper 100 shown in Figure 1 comprises a liquid-permeable surface layer 101, for example made of nonwoven or perforated plastic film, a backing layer 102, and an absorbent structure 103 enclosed between the surface layer and the backing layer.

The diaper is intended to surround the lower part of the abdomen of a wearer like a pair of absorbent underpants. To this end, it is shaped with a rear portion 104 and a front portion 105, and a crotch portion 106 which is located between the front portion 105 and the rear portion 104 and is intended during use to be arranged in the crotch of the wearer between the legs of the latter. In order that it is possible for the diaper to be fastened together in the desired pants-shape, tape flaps 107 are arranged close to the rear waist edge 108 of the diaper. During use, the tape flaps 107 are fastened to the front portion 105 of the diaper, close to the front waist edge 109, so that the diaper is held together around the waist of the wearer.

The diaper according to Figure 1 also comprises pretensioned elastic means 110 which can consist of elastic bands, thread-covered elastic threads, elastic foam or another suitable material. For the sake of simplicity, the elastic means 110 have in Figure 1 been shown in the stretched state. As soon as stretching stops, however, they contract and form elastic leg-bands of the diaper.

The liquid-permeable surface layer 101 is, for example, a nonwoven material or a perforated film, or a laminate thereof. Examples of polymers from which the liquid-permeable surface layer 101 can be made are polyethylene, polypropylene, polyester or copolymers thereof. In order that the liquid-permeable surface layer 101 will allow the discharged bodily fluid to pass through rapidly, it is common for the surface layer to be surfactant-coated and/or perforated. Another suitable material for use as a liquid-permeable surface layer is a layer of continuous fibres which are interconnected in a spot, line or patch pattern but are otherwise on the whole not attached to one another. The backing layer 102 is, for example, a plastic film, which is preferably breathable, a hydrophobic nonwoven layer or a laminate thereof. In the example shown in Figure 1, the absorbent structure 103 of the diaper is constructed from an upper liquid-receiving layer 111 and a lower liquid-spreading and storage layer 112. The lower liquid-spreading and storage layer 112 has a greater extent in the plane of the article than the upper liquid-receiving layer 111. The upper receiving layer 111 is located in the crotch portion 106 of the article, while the lower liquid-spreading and storage layer 112 extends over the rear portion 104, the crotch portion 106 and the front portion 105 of the article. The upper receiving layer 111 is to be capable of rapidly receiving large quantities of liquid in a short time, that is to say have a high instantaneous liquid absorption capacity, while the lower storage and spreading layer 112 is to be capable of draining liquid from the receiving layer 111 and spreading it in the storage and spreading layer 112. The upper receiving layer 111 in the absorbent structure 103 comprises the first superabsorbent material which is an odour-inhibiting and/or bacteria-inhibiting superabsorbent material which has a higher absorption rate than the second superabsorbent material. For example, the receiving layer consists of a fibrous structure made of natural fibres and/or synthetic fibres mixed with particles of the first superabsorbent material. The first superabsorbent material is, for example, a polyacrylate-based superabsorbent. According to one example, the receiving layer consists of a fibrous layer comprising polyacrylate-based particles or a polyacrylate-based coating bonded to the fibrous layer, the polyacrylate-based particles or the polyacrylate-based coating being bonded to the fibrous layer by acrylic acid monomers being sprayed onto the fibrous layer, after which the acrylic acid monomer is allowed to polymerize. An example of such a material is a nonwoven material made of, for example, polyester. Drops of acrylic acid monomer are sprayed onto the nonwoven material, the acrylic acid monomer then being allowed to polymerize. In addition to functioning as liquid-absorbing material, the polymerized polyacrylic acid particles formed also function as a bonding agent by virtue of the fact that the particles, by means of hydrogen bonds between oxygen in the carboxylic groups of the acrylic acid and hydrogen in the polyester waddling, maintain the structure in a compressed state. When wetting takes place, the hydrogen bonds present are broken, the material then expanding to its uncompressed thickness. The material subsequently swells further on account of the superabsorbent particles swelling when absorption of liquid takes place. This results in a material which is thin and relatively firmly compressed when it is dry but which has a great amount of free volume and high permeability when the material is then wetted. Another advantage of such a receiving layer is that the superabsorbent particles bind the liquid which is not drained by a storage layer, the risk of the surface next to the wearer becoming wet after initial wetting then being reduced. The superabsorbent material can of course also be bonded to the fibrous structure in ways other than that described above.

According to another example, the receiving layer is a superabsorbent foam, for example a polyacrylate-based foam. A polyacrylate-based superabsorbent foam is produced by a solution which consists of at least monomer, cross-linker, initiator and surfactant being saturated and pressurized with carbon dioxide in a vessel while being stirred. When the solution is removed from the vessel through a nozzle, the solution expands and a foamed structure is obtained. The foamed structure is then locked by polymerization and cross-linking being initiated by, for example, UV radiation. Finally, the material is compressed and dried. The receiving layer 111 can of course also consist of a mixed structure made of the first superabsorbent material and fibres, for example cellulose fluff pulp.

The second part of the absorbent structure constitutes the liquid-storing part of the structure and can consist of one or more layer(s), at least one of the layers consisting of the lower liquid-spreading and storage layer 112 and comprising the second superabsorbent material. In Figure 1, the second part of the absorbent structure consists of only the lower liquid-spreading and storage layer 112. For example, the lower liquid-spreading and storage layer 112 consists of a cellulose fibre structure mixed with particles of the second superabsorbent material. The second superabsorbent material is, for example, a polyacrylate-based superabsorbent with a degree of neutralization which is higher than 60%.

Figure 2 shows a cross section along the line II-II through the diaper 100 shown in Figure 1. The diaper 100 shown in Figure 2 therefore has a liquid-permeable surface layer 101, a backing layer 102, and an absorbent structure enclosed between the liquid-permeable surface layer 101 and the backing layer 102. The absorbent structure 103 of the diaper is constructed from an upper liquid-receiving layer 111 and a lower liquid-spreading and storage layer 112, which have been described in detail in the description of Figure 1.

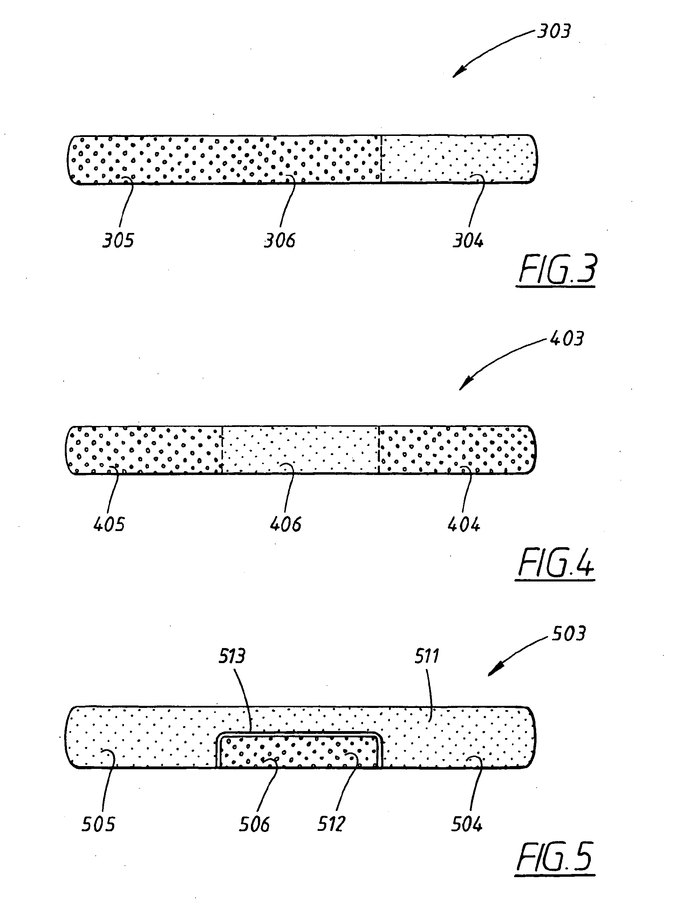

Figure 3 shows a section through an alternative absorbent structure 303 in the longitudinal direction of the absorbent structure 303. The absorbent structure 303 has a front portion 305, a rear portion 304, and a crotch portion 306 which is located between the front portion 305 and the rear portion 304 and is intended during use to be arranged in the crotch of the wearer between the legs of the latter. The rear portion 304 in the absorbent structure 303 comprises the first superabsorbent material. The front portion 305 and the crotch portion 306 comprise the second superabsorbent material. The rear portion 304 therefore has a superabsorbent material which is odour-inhibiting and/or bacteria-inhibiting and has a higher absorption rate than the superabsorbent material which is located in the crotch portion 306 and the front portion 305 of the absorbent structure. The absorbent structure 303 is intended primarily for use in incontinence pads used for people who are confined to bed. For such wearers, it is common that the discharged liquid runs backwards in the article. It is therefore important that the absorbent structure 303 has the capacity in the rear portion 304 rapidly to receive a large quantity of liquid which is absorbed in a short time.

Figure 4 shows a section through an alternative absorbent structure 403 in the longitudinal direction of the absorbent structure 403. The absorbent structure 403 has a front portion 405, a rear portion 404, and a crotch portion 406 which is located between the front portion 405 and the rear portion 404 and is intended during use to be arranged in the crotch of the wearer between the legs of the latter. Figure 4 shows the crotch portion 406 arranged centrally in the absorbent structure. However, this is not necessary for the invention, but the crotch portion 406 can alternatively be moved slightly forwards or backwards in the article. The crotch portion 406 can also occupy a greater or smaller part of the length of the article than is shown. The crotch portion 406 in the absorbent structure 403 comprises the first superabsorbent material. The front portion 405 and the rear portion 404 comprise the second superabsorbent material. The crotch portion 406 therefore has a superabsorbent material which is odour-inhibiting and/or bacteria-inhibiting, and has a higher absorption rate, than the superabsorbent material in the front portion 405 and the rear portion 404 of the absorbent structure. When the wearer sits, stands or walks, the wetting point is in the crotch portion 406 of the article. This means that the discharged liquid rapidly reaches the first superabsorbent material with the odour/bacteria-inhibiting effect.

Figure 5 shows a cross section of a further alternative absorbent structure 503 in the longitudinal direction of the absorbent structure. The absorbent structure 503 has a front portion 505, a rear portion 504, and a crotch portion 506 which is located between the front portion 505 and the rear portion 504 and is intended during use to be arranged in the crotch of the wearer between the legs of the latter. As in the case of the embodiment shown in Figure 4, both size and longitudinal positioning of the front portion 505 can vary within the scope of the invention. The crotch portion 506 in the absorbent structure 503 is constructed from an upper absorbent layer 511 and a lower absorbent layer 512. During use of an absorbent article, the upper absorbent layer 511 is located closer to the wearer, and the lower absorbent layer 512 is located further from the wearer.

The upper absorbent layer 511 is made from cellulose fluff pulp mixed with the first superabsorbent material, that is to say the odour-inhibiting and/or bacteria-inhibiting superabsorbent material. The lower absorbent layer 512 comprises the second superabsorbent material. For example, the lower absorbent layer 512 is made from cellulose fluff pulp mixed with the second superabsorbent material. The lower absorbent layer 512 is separated from direct contact with the upper absorbent layer 511 by virtue of a thin layer 513 being located between the lower absorbent layer 512 and the upper absorbent layer 511. The thin layer 513 therefore encapsulates the lower absorbent layer 512. This means that the time before the liquid reaches the lower absorbent layer 512 and thus also the second superabsorbent material is extended. In this way, the activation time of the second superabsorbent material is retarded. The thin layer 513 is, for example, a tissue layer, a nonwoven material, a perforated plastic film or a laminate thereof.

### Example 1 - Measurement of absorption rate in superabsorbent material

The absorption rate of three different polyacrylate-based superabsorbent materials was measured using the "Free swell rate" method. Two of the superabsorbent materials were manufactured by BASF and are .called Hysorb C7110 and Hysorb B7160 respectively. The third superabsorbent material was manufactured by Dow and is called Drytech S230R. The pH value of the superabsorbent material called Hysorb C7110 is 4.5, the pH value of the superabsorbent material called Hysorb B7160 is 6.0, and the pH value of the superabsorbent material called Drytech S230R is 5.9. For measuring the pH of the superabsorbent materials, use was made of the EDANA method 400.1-99.

The absorption rate was measured in three different particle size ranges. This means that nine different measurements were performed.

The principle of Free swell rate measurement is to allow a superabsorbent material to absorb a given quantity of liquid. The time from the moment when the liquid is added to the moment when the superabsorbent material has completely absorbed the liquid is measured.

Measurement is performed as follows:
1.0 gram of the superabsorbent material is weighed out and placed in a 25 ml beaker. The superabsorbent material is distributed uniformly over the bottom surface of the beaker. 20 ml of liquid are then added. The liquid used is 0.9% by weight NaCl solution. The liquid is suitably added with a pipette. The timing is started directly the liquid has been added. The timing is stopped when all the liquid has been absorbed.

### Result

| Material (g/g/sec) | Particle size (µm) | Absorption rate |
|---|---|---|
| Drytech S230R | 150-300 | 0.45 |
| Drytech S230R | 300-500 | 0.24 |
| Drytech S230R | 500-710 | 0.15 |
| | | |
| Hysorb C7110 | 150-300 | 0.48 |
| Hysorb C7110 | 300-500 | 0.31 |
| HysorbC7110 | 500-710 | 0.18 |
| | | |
| Hysorb B7160 | 150-300 | 0.54 |
| Hysorb B7160 | 300-500 | 0.23 |
| Hysorb B7160 | 500-710 | 0.15 |

The result shows that superabsorbent materials with a small particle size absorb liquid more rapidly than superabsorbent materials with a large particle size. The superabsorbent material which is called Hysorb C7110 and has a small particle size is therefore an example of the first superabsorbent material, that is to say the odour-inhibiting and/or bacteria-inhibiting superabsorbent material. Furthermore, the superabsorbent materials called Hysorb B7160 and Drytech S230R which have a slower absorption rate than superabsorbent Hysorb C7110 are examples of a second superabsorbent material.

Other methods can also be used for measuring the absorption rate. In order for it to be possible to determine the difference in the absorption rate between the first superabsorbent material and the second superabsorbent material, however, it is important that the same method is used for measuring the absorption rate of both the first and the second superabsorbent material. For example, a method can be used in which the absorption rate during the first five seconds is measured.

### Example 2 - Measurement of pH in an absorbent structure

An absorbent structure with a diameter of roughly 50 mm was produced according to a slightly modified test specimen forming procedure according to SCAN C 33:80. Fluff pulp and superabsorbent material were weighed out, and a uniform mixture of fluff pulp and superabsorbent material was introduced into an air flow with a negative pressure of roughly 85 mbar and guided through a tube with a diameter of 5 cm provided with a metal net at the bottom on which a thin tissue had been placed. The mixture of fluff pulp and superabsorbent material accumulated on the tissue on the metal net and formed the absorbent structure. The absorbent structure was weighed and compressed to a bulk of between 6 and 12 cm³/g. The absorbent structure tested had a total weight of 1 gram. The absorbent structure contained a partly neutralized superabsorbent material with a pH of 4.2. The fluff pulp was a chemithermomechanical cellulose pulp with a pH of 5.8. The proportion of superabsorbent material was 15% by weight and the proportion of fluff pulp was 85% by weight. Test liquid 1 was 0.9% salt solution, test liquid 2 was synthetic urine with the composition 2 g/l KCl, 2 g/l Na₂SO₄, 0.85 g/l (NH₄)H₂PO₄, 0.15 g/l (NH₄)2HPO₄, 0.19 g/l CaCl₂ and 0.23 g/l MgCl₂. The pH of this mixture was 6.0-6.4. Test liquid 3 was synthetic urine containing the following substances: KCI, NaCl, MgSO₄, KH₂PO₄, NH₂CONH₂. The pH of this mixture was 6.0-6.5.

10 ml test liquid was added to the absorbent structure. The absorbent structure was then allowed to swell for 30 minutes. Then, the pH in the absorbent structure was measured by means of a surface electrode, Flatbottnad [flat-bottomed] Metrohm pH meter, Beckman θ12 or θ72. Parallel measurements were performed on at least two different absorbent structures. The pH was measured at 10 points on each absorbent structure, and the mean was calculated.

### Result

| | Test liquid 1 | Test liquid 2 | Test liquid 3 |
|---|---|---|---|
| pH | 4.29 | 4.42 | |
| | 4.54 | | |

The pH measured in the absorbent structure therefore lies within the pH range 3.5-4.9. With a content of 15% by weight of the odour-inhibiting and/or bacteria-inhibiting superabsorbent material, a pH is therefore obtained in the absorbent structure which lies within the pH range 3.5-4.9. In order to obtain a thin absorbent structure, it is common to use a content of superabsorbent material higher than 15% by weight. The rest of the superabsorbent material does not therefore have to have any pH-lowering effect and can therefore advantageously consist of the second superabsorbent material.

## Claims

1. Absorbent article such as a diaper, an incontinence pad, a sanitary towel or the like, the article having a liquid-permeable upper surface and comprising an absorbent structure which comprises at least a first superabsorbent material and a second superabsorbent material, **characterized in that** the first superabsorbent material is an odour-inhibiting and/or bacteria-inhibiting superabsorbent material, and **in that** the first superabsorbent material has a higher absorption rate than the second superabsorbent material.

2. Absorbent article according to Claim 1, **characterized in that** the first superabsorbent material has a degree of neutralization of from 20 to 60%.

3. Absorbent article according to Claim 1 or 2, **characterized in that** the absorbent structure has a pH in the wet state during use which lies in the range 3.5-4.9.

4. Absorbent article according to any one of Claims 1-3, **characterized in that** the second superabsorbent material has a degree of neutralization which is higher than 60%.

5. Absorbent article according to any one of the preceding claims, **characterized in that** the first superabsorbent material has a greater specific surface per gram of superabsorbent material than the second superabsorbent material.

6. Absorbent article according to preceding claims, **characterized in that** the first superabsorbent material and the second superabsorbent material are in particle form, the first superabsorbent material having a smaller particle size then the second superabsorbent material.

7. Absorbent article according to any one of the preceding claims, **characterized in that** at least one superabsorbent material is treated in such a way that a difference in the absorption.rate of the superabsorbent materials is obtained.

8. Absorbent article according to any one of the preceding claims, **characterized in that** the second superabsorbent material is surrounded by a covering which is only slowly dissolved in and/or penetrated by the liquid which is to be absorbed, so that the superabsorbent material does not start to absorb liquid and swell to any great extent until the covering has been dissolved and/or penetrated by the liquid.

9. Absorbent article according to any one of the preceding claims, the article having in the longitudinal direction a front portion, a rear portion and a crotch portion, **characterized in that** the first superabsorbent material is mainly located in the crotch portion and the second superabsorbent material is mainly located in the front portion and the rear portion.

10. Absorbent article according to any one of Claims 1-7, **characterized in that** the first superabsorbent material is mainly located in the rear portion and the second superabsorbent material is mainly located in the front portion and the crotch portion.

11. Absorbent article according to any one of the preceding claims, the absorbent structure comprising a first part which faces the liquid-permeable surface of the article and a second part which faces away from the liquid-permeable surface of the article, **characterized in that** the first superabsorbent material is mainly located in the first part and the second superabsorbent material is mainly located in the second part.

12. Absorbent article according to any one of the preceding claims, **characterized in that** the first superabsorbent material is located in an absorbent insert.

## Patentansprüche

1. Absorbierender Gegenstand, wie eine Windel, eine Inkontinenzbinde, eine Damenbinde oder dergleichen, wobei der Gegenstand eine flüssigkeitsdurchlässige obere Oberfläche aufweist und eine absorbierende Struktur umfaßt, die mindestens ein erstes Superabsorbermaterial und ein zweites Superabsorbermaterial umfaßt, **dadurch gekennzeichnet, daß** das erste Superabsorbermaterial ein geruchshemmendes und/oder bakterienhemmendes Superabsorbermaterial ist und das erste Superabsorbermaterial eine höhere Absorptionsgeschwindigkeit aufweist als das zweite Superabsorbermaterial.

2. Absorbierender Gegenstand gemäß Anspruch 1, **dadurch gekennzeichnet, daß** das erste Superabsorbermaterial einen Neutralisationsgrad von 20 bis 60 % aufweist.

3. Absorbierender Gegenstand gemäß Anspruch 1 oder 2, **dadurch gekennzeichnet, daß** die absorbierende Struktur einen pH-Wert im nassen Zustand während der Verwendung aufweist, der im Bereich von 3,5 bis 4,9 liegt.

4. Absorbierender Gegenstand gemäß mindestens einem der Ansprüche 1 bis 3, **dadurch gekennzeichnet, daß** das zweite Superabsorbermaterial einen Neutralisationsgrad aufweist, der höher als 60 % ist.

5. Absorbierender Gegenstand gemäß mindestens einem der vorhergehenden Ansprüche, **dadurch gekennzeichnet, daß** das erste Superabsorbermaterial eine größere spezifische Oberfläche pro Gramm des Superabsorbermaterials als das zweite Superabsorbermaterial aufweist.

6. Absorbierender Gegenstand gemäß mindestens einem der vorhergehenden Ansprüche, **dadurch gekennzeichnet, daß** das erste Superabsorbermaterial und das zweite Superabsorbermaterial in Teilchenform vorliegen, wobei das erste Superabsorbermaterial eine kleinere Teilchengröße als das zweite Superabsorbermaterial aufweist.

7. Absorbierender Gegenstand gemäß mindestens einem der vorhergehenden Ansprüche, **dadurch gekennzeichnet, daß** mindestens ein Superabsorbermaterial so behandelt ist, daß ein Unterschied in der Absorptionsgeschwindigkeit der Superabsorbermaterialien erhalten wird.

8. Absorbierender Gegenstand gemäß mindestens einem der vorhergehenden Ansprüche, **dadurch gekennzeichnet, daß** das zweite Superabsorbermaterial von einer Abdeckung umgeben ist, die sich nur langsam in der zu absorbierenden Flüssigkeit löst und/oder von dieser durchdrungen wird, so daß das Superabsorbermaterial nicht beginnt, Flüssigkeit zu absorbieren und auf ein größeres Ausmaß anzuschwellen, bevor die Abdeckung gelöst und/oder von der Flüssigkeit durchdrungen wurde.

9. Absorbierender Gegenstand gemäß mindestens einem der vorhergehenden Ansprüche, wobei der Gegenstand in Längsrichtung ein Vorderteil, ein Hinterteil und ein Schritteil aufweist, **dadurch gekennzeichnet, daß** sich das erste Superabsorbermaterial hauptsächlich im Schritteil befindet und sich das zweite Superabsorbermaterial hauptsächlich im Vorderteil und im Hinterteil befindet.

10. Absorbierender Gegenstand gemäß mindestens einem der Ansprüche 1 bis 7, **dadurch gekennzeichnet, daß** sich das erste Superabsorbermaterial hauptsächlich im Hinterteil befindet und sich das zweite Superabsorbermaterial hauptsächlich im Vorderteil und im Schritteil befindet.

11. Absorbierender Gegenstand gemäß mindestens einem der vorhergehenden Ansprüche, wobei die absorbierende Struktur einen ersten Teil umfaßt, der die flüssigkeitsdurchlässige Oberfläche des Gegenstands belegt, und einen zweiten Teil umfaßt, der abgewendet von der flüssigkeitsdurchlässigen Oberfläche des Gegenstands liegt, **dadurch gekennzeichnet, daß** sich das erste Superabsorbermaterial hauptsächlich im ersten Teil befindet und sich das zweite Superabsorbermaterial hauptsächlich im zweiten Teil befindet.

12. Absorbierender Gegenstand gemäß mindestens einem der vorhergehenden Ansprüche, **dadurch gekennzeichnet, daß** sich das erste Superabsorbermaterial in einer absorbierenden Einlage befindet.

## Revendications

1. Article absorbant tel qu'une couche-culotte, une couche d'incontinence, une serviette hygiénique ou autres, l'article possédant une surface externe perméable aux liquide et comprenant une structure absorbante qui comprend au moins un premier matériau super-absorbant et un second matériau super-absorbant, **caractérisé en ce que** le premier matériau super-absorbant est un matériau super-absorbant empêchant le développement des odeurs et/ou des bactéries, et **en ce que** le premier matériau super-absorbant possède une vitesse d'absorption plus élevée que celle du second matériau super-absorbant.

2. Article absorbant selon la revendication 1, **caractérisé en ce que** le premier matériau super-absorbant possède un degré de neutralisation allant de 20 à 60%.

3. Article absorbant selon la revendication 1 ou 2, **caractérisé en ce que** la structure absorbante possède un pH à l'état mouillé au cours de l'utilisation qui est compris dans la plage de 3,5 à 4,9.

4. Article absorbant selon l'une quelconque des revendications 1 à 3, **caractérisé en ce que** le second matériau super-absorbant possède un degré de neutralisation qui est supérieur à 60%.

5. Article absorbant selon l'une quelconque des revendications précédentes, **caractérisé en ce que** le premier matériau super-absorbant possède une surface spécifique par gramme de matériau super-absorbant plus élevée que celle du second matériau super-absorbant.

6. Article absorbant selon les revendications précédentes, **caractérisé en ce que** le premier matériau super-absorbant et le second matériau super-absorbant sont sous forme de particules, le premier matériau super-absorbant possédant une taille de particule plus faible que celle du second matériau super-absorbant.

7. Article absorbant selon l'une quelconque des revendications précédentes, **caractérisé en ce qu'**au moins un matériau super-absorbant est traité de manière à obtenir une différence de vitesse d'absorption entre les matériaux super-absorbants.

8. Article absorbant selon l'une quelconque des revendications précédentes, **caractérisé en ce que** le second matériau super-absorbant est entouré d'un revêtement qui est seulement lentement dissous dans et/ou pénétré par le liquide qui doit être absorbé, de sorte que le matériau super-absorbant ne commence pas à absorber le liquide et à gonfler dans une quelconque mesure importante avant que le revêtement n'ait été dissous et/ou pénétré par le liquide.

9. Article absorbant selon l'une quelconque des revendications précédentes, l'article possédant dans la direction longitudinale une partie à positionner devant, une partie à positionner derrière et une partie pour l'entrejambe, **caractérisé en ce que** le premier matériau super-absorbant est principalement situé dans la partie pour l'entrejambe et le second matériau super-absorbant est principalement situé dans la partie à positionner devant et dans la partie à positionner derrière.

10. Article absorbant selon l'une quelconque des revendications 1 à 7, **caractérisé en ce que** le premier matériau super-absorbant est principalement situé dans la partie à positionner derrière et le second matériau super-absorbant est principalement situé dans la partie à positionner devant et dans la partie pour l'entrejambe.

11. Article absorbant selon l'une quelconque des revendications précédentes, la structure absorbante comprenant une première partie qui est orientée face à la surface perméable au liquide de l'article et une seconde partie qui est orientée à l'opposé de la surface perméable au liquide de article, **caractérisé en ce que** le premier matériau super-absorbant est principalement situé dans la première partie et le second matériau super-absorbant est principalement situé dans la seconde partie.

12. Article absorbant selon l'une quelconque des revendications précédentes, **caractérisé en ce que** le premier matériau super-absorbant est situé dans une garniture absorbante.
